# FASCICULE DE BREVET EUROPEEN

(11) **EP 3 464 511 B1**
(45) Date de publication et mention de la délivrance du brevet: **07.07.2021**
(21) Numéro de dépôt: 17720819.6
(22) Date de dépôt: 03.05.2017
(51) Int. Cl.: C10G 1/00, B01J 23/06, B01J 23/08, B01J 23/745, B01J 29/08, B01J 29/18, B01J 29/40, B01J 29/65, C10B 53/02, C10G 1/08, C12P 7/02, C12P 7/52, C12R 1/00, B01J 29/70, C10B 57/06, C12P 7/04, C12P 7/06, C12P 7/16, C12P 7/18

(54) **PROCÉDÉ DE PRODUCTION DE BTX PAR PYROLYSE CATALYTIQUE À PARTIR DE BIOMASSE SANS RECYCLE DE COMPOSÉS OXYGÉNÉS**
VERFAHREN ZUR HERSTELLUNG VON BTX DURCH KATALYTISCHE PYROLYSE AUS BIOMASSE OHNE RÜCKFÜHRUNG VON OXYGENIERTEN VERBINDUNGEN
PROCESS FOR PRODUCING BTX BY CATALYTIC PYROLYSIS FROM BIOMASS WITHOUT RECYCLING OXYGENATED COMPOUNDS

(30) Priorité: 31.05.2016 FR 1654894
(43) Date de publication de la demande: 10.04.2019
(73) Titulaire: IFP Energies nouvelles, 92852 Rueil-Malmaison (FR)
(72) Inventeur: FEUGNET, Frederic, 69004 Lyon (FR); LOPES FERREIRA, Nicolas, 28210 Croisilles (FR); KASZTELAN, Slavik, 69006 Lyon (FR)
(74) Mandataire: IFP Energies nouvelles
(86) Numéro de dépôt international: PCT/EP2017/060548
(87) Numéro de publication internationale: WO 2017/207201

(56) Documents cités:
- US-A1- 2014 107 306
- US-A1- 2014 134 686
- YU-TING CHENG ET AL: "Production of Renewable Aromatic Compounds by Catalytic Fast Pyrolysis of Lignocellulosic Biomass with Bifunctional Ga/ZSM-5 Catalysts", ANGEWANDTE CHEMIE INTERNATIONAL EDITION, vol. 51, no. 6, 27 décembre 2011 (2011-12-27), pages 1387-1390, XP055068876, ISSN: 1433-7851, DOI: 10.1002/anie.201107390
- TORREN R CARLSON ET AL: "Aromatic Production from Catalytic Fast Pyrolysis of Biomass-Derived Feedstocks", TOPICS IN CATALYSIS, KLUWER ACADEMIC PUBLISHERS-PLENUM PUBLISHERS, NE, vol. 52, no. 3, 14 janvier 2009 (2009-01-14), pages 241-252, XP019689593, ISSN: 1572-9028

## Description

### Domaine technique

Les intermédiaires chimiques sont normalement produits à partir de ressources fossiles comme le pétrole, le gaz naturel ou le charbon dans des procédés multi-étapes. Afin de remplacer ou de compléter la production d'intermédiaires chimiques à partir de ressources fossiles, il est nécessaire de développer des procédés traitant des charges non fossiles, à savoir la biomasse. La présente invention concerne un procédé de production d'intermédiaires chimiques et ou de base pour carburant et en particulier de BTX et d'alcools à partir de la biomasse.

### Art antérieur

Le brevet US8277643 décrit un procédé de production d'aromatiques, de bio carburant et d'oléfines par pyrolyse catalytique de biomasse en présence d'un catalyseur zéolitique tel que la ZSM-5. Le procédé est mis en œuvre à une température au moins supérieure à 300°C, à une pression comprise entre 1 et 4 atm, à une vvh comprise entre 0,01 et 10 h⁻¹. Le procédé se caractérise également par un temps de résidence de la biomasse dans le réacteur compris entre 2 secondes et 5 minutes.

La demande de brevet WO2009/111026 décrit de manière plus précise le type de catalyseur qui peut être utilisé dans l'étape de pyrolyse catalytique et en particulier des catalyseurs zéolitiques qui peuvent être dopés au fer, au gallium ou au zinc.

La demande de brevet WO2011/031320 décrit un procédé de production d'aromatiques, de bio carburant et d'oléfines par pyrolyse catalytique de biomasse en présence d'un catalyseur zéolitique, dans lequel les produits obtenus par pyrolyse sont séparés de manière à obtenir une fraction comprenant des oléfines qui est ensuite recyclés dans le réacteur de pyrolyse.

US 2014/0107306 divulgue un procédé de production de BTX par catalyse pyrolytique.

La demanderesse a ainsi découvert que la mise en œuvre d'un procédé de production d'une coupe BTX par pyrolyse catalytique de biomasse lignocellulosique en présence d'un flux de composés oxygénés spécifiques permettait l'obtention d'un rendement amélioré en BTX par rapport aux procédés de l'art antérieur.

### Résumé et intérêt de l'invention

L'invention concerne un procédé de production de BTX et d'alcools à partir de biomasse tel que défini dans les revendications.

Dans toute la suite du texte, on entend par coupe BTX, une fraction comprenant un mélange de Benzène, de Toluène et de Xylènes (ortho, méta, para).

Un avantage de la présente invention est de fournir un procédé intégré de production de BTX avec un rendement élevé par pyrolyse catalytique de biomasse, suivie d'une étape fermentaire de conversion d'une partie de l'effluent gazeux gazeux de pyrolyse comprenant du CO et du CO₂, ledit procédé permettant également la production d'un flux de composés oxygénés et en particulier d'alcool et de préférence d'éthanol.

Un autre avantage de ce mode de réalisation de la présente invention réside également dans la valorisation de la purge de ladite fraction gazeuse de recycle comprenant du CO et du CO₂ produit par pyrolyse catalytique dans une étape de fermentation pour produire au moins un composé oxygéné tel que revendiqué et de préférence l'éthanol, permettant donc d'améliorer très nettement la rentabilité du procédé selon l'invention.
Un autre avantage de ce mode de réalisation de la présente invention réside donc dans l'optimisation de la valorisation du carbone issu de la biomasse, en particulier en intermédiaire chimique tels que les BTX, mais également en base pour biocarburant.

Un autre avantage de la présente invention est de fournir un procédé optimisé de production d'une coupe BTX en ce que l'effluent gazeux comprenant du CO et du CO₂ produit par pyrolyse catalytique et utilisé comme charge de l'étape fermentaire est soutiré de l'étape de pyrolyse catalytique à une température et une pression compatible à son utilisation directe dans une étape de fermentation, sans étape de compression intermédiaire. L'effluent gazeux comprenant du CO et du CO₂ produit par pyrolyse catalytique présente également une composition compatible à son utilisation dans une étape fermentaire.

### Description détaillée de l'invention

Conformément à l'invention, le procédé de production de BTX et d'alcools à partir de biomasse une étape a) de pyrolyse catalytique de ladite biomasse dans un réacteur en lit fluidisé produisant un effluent gazeux de pyrolyse.

### Etape a) de pyrolyse catalytique

Des exemples de réacteurs à lit fluidisé peuvent être trouvés dans Howard, R. J. (1989). «Principes de la technologie à lit fluidisé et des applications." New York, Adam Higler de N.Y.; Tavoulareas, S. (1991) Combustion Technology. ** Annual Reviews Inc ** 16, 25-27. et Trambouze, P., et Euzen, J. (2004). "Réacteurs chimiques: De la conception à l'opération." (R. Bonormo, Trans.). Paris: Editions Technip.
Le réacteur en lit fluidisé utilisé dans le procédé selon l'invention opère de préférence en régime turbulent, c'est-à-dire dans une gamme de vitesse de gaz superficielle comprise entre 0,5 et 0,8 m/s. Par vitesse de gaz superficielle, on entend le débit volumique de gaz rapporté à la surface du réacteur.
Cette mise en œuvre en lit turbulent est privilégiée par rapport à la technologie dite « riser » usuellement utilisée pour le FCC dans la mesure où les rendements en coke et en biomasse non totalement convertie usuellement dénommée « char » dans la technologie de FCC sont importants et que par conséquent la concentration en coke sur le catalyseur est élevée. Grâce à la mise en œuvre proposée, le bilan thermique de l'étape de pyrolyse catalytique est découplée en réglant le temps de résidence du catalyseur dans le réacteur via le débit de soutirage de ce dernier vers le régénérateur, permettant ainsi de limiter le pourcentage de coke sur le catalyseur et donc de limiter la température du régénérateur dans une gamme opérable.

Selon l'invention, la charge utilisée dans la présente invention est la biomasse.
De préférence, la charge est la biomasse lignocellulosique ou un ou plusieurs constituants de biomasse lignocellulosique choisis dans le groupe formé par la cellulose, l'hémicellulose et/ou la lignine.
La biomasse lignocellulosique peut être constituée de bois, de déchets agricole ou de déchets végétaux. D'autres exemples non limitatifs de matière biomasse lignocellulosique sont les résidus d'exploitation agricole (paille, tige de maïs...), les résidus d'exploitation forestière (produits de première éclaircie), les produits d'exploitation forestière, les cultures dédiées (taillis à courte rotation), les résidus de l'industrie agro-alimentaire, les déchets organiques ménagers, les déchets des installations de transformation du bois, les bois usagés de construction, du papier, recyclé ou non.
La biomasse lignocellulosique peut aussi provenir de sous-produits de l'industrie papetière comme la lignine Kraft, ou les liqueurs noires issues de la fabrication de pâte à papier.
La biomasse lignocellulosique peut avantageusement subir au moins une étape de prétraitement avant son introduction dans le procédé selon l'invention. De préférence, la biomasse est broyée et séchée, jusqu'à ce que la granulométrie désirée soit obtenue. Une charge présentant un diamètre de particules compris entre 0,3 et 0,5 mm peut avantageusement être obtenue. Typiquement, la taille des particules de la biomasse lignocellulosique à pyrolyser est une taille de particules suffisante pour passer à travers un tamis de 1 mm jusqu'à une taille de particules suffisante pour passer à travers un tamis de 30 mm.

De préférence, la biomasse à pyrolyser est avantageusement chargée dans un compartiment d'entraînement ou transport pneumatique de manière à être entrainée dans le lit fluidisé par un fluide d'entrainement. De préférence, le fluide d'entrainement utilisé est de l'azote gazeux. Cependant, il est également envisagé que d'autres fluides d'entrainement non oxydants peuvent être utilisés. De préférence, le gaz de pyrolyse produit au cours du processus peut être recyclé et utilisé comme fluide d'entraînement. Ledit gaz de pyrolyse est principalement constitué d'un effluent gazeux, incondensable, comprenant au moins du monoxyde de carbone (CO), et du dioxyde de carbone (CO₂) et comprenant également avantageusement des oléfines légères comprenant de 2 à 4 atomes de carbone, produites lors de l'étape a). De cette manière, le coût de la réalisation de la pyrolyse peuvent être considérablement réduits. La biomasse peut être chargée dans une trémie d'alimentation ou un autre dispositif qui permet d'amener la biomasse dans le compartiment d'entraînement dans une quantité appropriée. De cette manière, une quantité constante de biomasse est délivrée dans le compartiment d'entraînement.

Le fluide d'entrainement transporte avantageusement la biomasse à partir du compartiment d'entraînement dans le lit fluidisé à travers un tube d'alimentation. Typiquement, le tube d'alimentation est refroidi pour maintenir la température de la biomasse à un niveau requis avant son entrée dans le lit fluidisé. Le tube d'alimentation peut être refroidi par chemisage du tube, typiquement avec une chemise refroidie par air ou refroidi par liquide. Cependant, il est également envisagé que le tube d'alimentation ne soit pas refroidi.

Selon l'invention, l'étape a) de pyrolyse catalytique opère en présence d'un catalyseur. De préférence, ladite étape a) opère en présence d'un catalyseur zéolithique comprenant et de préférence constitué par au moins une zéolithe choisie parmi la ZSM-5, la ferriérite, la zéolithe Béta, la zéolithe Y, la mordenite, la ZSM-23, la ZSM-57, la EU-1, la ZSM-11 et de préférence le catalyseur est un catalyseur comprenant uniquement de la ZSM-5. La zéolithe utilisée dans la catalyseur mis en œuvre dans l'étape a) de pyrolyse catalytique peut avantageusement être dopée de préférence par un métal choisi parmi le Fer, le Gallium, le Zinc et le Lanthane.

En fonction du catalyseur utilisé et les produits de réaction désirés, la température mise en œuvre dans l'étape a) de pyrolyse catalytique peut être ajustée. Dans certains modes de réalisation, l'étape de pyrolyse catalytique est mise en œuvre à une température comprise entre 400 et 1000°C, de préférence entre 400 et 650 °C, de préférence entre 450 et 600 °C et de préférence entre 450 et 590 °C. en particulier, c'est le catalyseur provenant de l'étape de régénération qui permet d'assurer ces gammes de température du réacteur. L'étape a) de pyrolyse est également avantageusement mise en œuvre a une pression absolue comprise entre 0,1 et 0,5 MPa et à une vvh comprise entre 0,01 et 10 h⁻¹, de préférence entre 0,01 et 5 h⁻¹, de manière préférée entre 0,1 et 3h⁻¹ et de manière très préférée entre 0,1 et 3h⁻¹.

Dans ces conditions, la biomasse va tout d'abord subir dans le réacteur une pyrolyse rapide en contactant le catalyseur chaud provenant du régénérateur qui dans cette étape joue le rôle de vecteur thermique. Les gaz résultants de cette pyrolyse vont ensuite réagir sur le catalyseur qui cette fois joue son rôle de catalyseur permettant de catalyser les réactions produisant les intermédiaires chimiques recherchés.

Conformément à l'invention, les produits obtenus à l'issue de l'étape a) de pyrolyse catalytique sont avantageusement récupérés sous forme d'un effluent gazeux de pyrolyse comprenant au moins une partie de la coupe BTX.

### Etape b) de séparation

Conformément à l'invention, le procédé comprend une étape b) de séparation dudit effluent gazeux de pyrolyse en au moins une fraction BTX et un effluent gazeux comprenant au moins du monoxyde de carbone (CO) et du dioxyde de carbone (CO₂).
Avantageusement, outre la fraction liquide BTX et l'effluent gazeux, incondensable, comprenant au moins du monoxyde de carbone (CO) et du dioxyde de carbone (CO₂), l'étape b) de séparation permet également de séparer une coupe liquide comprenant majoritairement des composés présentant un nombre d'atomes de carbone supérieur à 9, c'est-à-dire au moins 50% poids de composés en C9+ et de l'eau.
Ledit effluent gazeux, incondensable, comprenant au moins du monoxyde de carbone (CO), et du dioxyde de carbone (CO₂) comprend également avantageusement des oléfines légères comprenant de 2 à 4 atomes de carbone.

Le catalyseur coké ainsi que la biomasse non convertie usuellement dénommée « char » sont avantageusement soutirés du réacteur et envoyés de préférence dans un stripper de manière à éliminer les hydrocarbures potentiellement adsorbés et ainsi éviter leur combustion dans le régénérateur et ce par mise en contact avec un gaz choisi parmi la vapeur d'eau, un gaz inerte tel que par exemple de l'azote et au moins une partie de la coupe gazeuse incondensable riche en CO et CO₂ issue du fractionnement de l'effluent gazeux issu de l'étape de pyrolyse catalytique.

Ledit catalyseur coké et le char, éventuellement strippés, sont avantageusement envoyés dans un régénérateur où coke et char sont brûlés par ajout d'air ou d'oxygène.

Le catalyseur ainsi régénéré est avantageusement recyclé dans le réacteur de l'étape de pyrolyse catalytique afin de subir un autre cycle.

L'étape de pyrolyse catalytique du procédé selon l'invention permet la production d'au moins 10% poids et de préférence d'au moins 15 % poids d'aromatique par rapport à la masse totale des produits de réaction obtenus, avec une sélectivité d'au moins 65% et de préférence d'au moins 70% en BTX.

Le procédé selon l'invention comprenant au moins une étape de pyrolyse catalytique d'une charge biomasse produit donc un effluent gazeux comprenant au moins une fraction liquide BTX et un effluent gazeux comprenant au moins du monoxyde de carbone et du dioxyde de carbone.
Le procédé permet également d'obtenir en plus de la coupe BTX, une fraction liquide plus lourde, majoritairement aromatique appelée « coupe C9+ » qui peut avantageusement être valorisée dans un procédé extérieur au procédé selon l'invention.

### Etape c) de recycle

Conformément à l'invention, le procédé comprend une étape c) de recycle d'au moins une partie dudit effluent gazeux comprenant au moins du monoxyde de carbone et du dioxyde de carbone dans le réacteur de ladite étape a).

De préférence, le recycle d'au moins une partie dudit effluent gazeux comprenant au moins du monoxyde de carbone et du dioxyde de carbone dans le réacteur de l'étape de pyrolyse catalytique est réalisé via un compresseur. Ce flux gazeux sert alors de fluide d'entrainement de la charge dans ledit réacteur.

Dans ce cas, une purge dudit effluent gazeux de recycle est inévitable.

### Etape d) de purge

Conformément à l'invention, le procédé comprend une étape d) de purge dudit effluent gazeux recyclé selon l'étape c) pour produire un effluent de purge.

De préférence, ladite purge dudit effluent gazeux recyclé est réalisée soit en amont, soit en aval dudit compresseur placé sur la boucle de recycle.

### Etape fermentaire e)

L'effluent de purge de la fraction gazeuse de recycle comprend généralement une teneur en monoxyde de carbone (CO) avantageusement comprise entre 40 et 60% massique et une teneur en dioxyde de carbone (CO₂) avantageusement comprise entre 20 et 40% massique.

Par ailleurs, ledit effluent de purge comprend également généralement de l'hydrogène, des gaz légers en C1-C4, des oléfines et peut également contenir une faible teneur en impuretés aromatiques telles que par exemple en benzène et en toluène.

La teneur en impuretés aromatiques peut avantageusement être diminuée en jouant sur les conditions opératoires de l'étape de pyrolyse catalytique ainsi que sur le taux de recycle renvoyé au réacteur.

L'effluent gazeux comprenant du CO et du CO₂ produit par pyrolyse catalytique présente une composition compatible à son utilisation dans une étape fermentaire.

Par ailleurs, la partie dudit effluent de purge de l'étape d) comprenant du monoxyde de carbone (CO), et du dioxyde de carbone (CO₂) utilisée comme charge de l'étape fermentaire e) est soutirée de l'étape a) de pyrolyse catalytique à une température et une pression compatible à son utilisation directe dans une étape de fermentation. Le procédé selon l'invention peut nécessiter de comprimer ou décomprimer la dite purge pour permettre un meilleur fonctionnement de l'étape fermentaire. Dans un mode préféré, le procédé selon l'invention ne comprend pas d'étape de compression ni de chauffe dudit effluent de purge de l'étape d) comprenant du monoxyde de carbone (CO), et du dioxyde de carbone (CO₂) pour l'amener à des conditions opératoires compatibles avec une étape fermentaire.

La partie dudit effluent de purge de l'étape d) comprenant du monoxyde de carbone (CO), et du dioxyde de carbone (CO₂) utilisée comme charge de l'étape fermentaire e) est généralement soutirée à une température comprise entre 20 et 60°C et à une pression comprise entre 0,1 et 0,5 MPa.

L'étape fermentaire e) est avantageusement mise en œuvre en présence d'au moins un microorganisme également appelé souche acétogène.
Dans toute la suite du texte, les termes «fermentation», « étape fermentaire », ou « réaction de fermentation » se rapporte à la conversion des gaz H₂, CO et/ou CO₂ et englobent à la fois la phase de croissance du microorganisme fermentaire et de la phase de production des molécules d'intérêt, telles que les alcools, les acides, les acides alcools et/ou les acides carboxyliques par ce microorganisme.

En effet, la capacité de certains microorganismes à se développer sur des substrats gazeux de type monoxide de carbone (CO), dioxide de carbone (CO₂) et/ou hydrogène (H₂) en tant que seule source de carbone a été découvert dès 1903. Un grand nombre d' organismes anaérobies, plus particulièrement les organismes dits « acétogènes », possèdent cette capacité de métaboliser le CO et/ou le couple CO₂/H₂ pour produire diverses molécules finales d'intérêt comme l'acétate, le butyrate, l'éthanol et/ou le n-butanol.
Les microorganismes capables de réaliser ce processus fermentaire sont principalement issus du genre *Clostridium,* mais d'autres microorganismes, comme ceux issus par exemple des genres *Acetobacteria, Butyribacterium, Desulfobactrium, Moorella, Oxobacter* ou encore *Eubacteria* peuvent également être utilisés pour réaliser ce processus fermentaire.
Les microorganismes sont donc choisies de manière à orienter la production des produits désirés lors de l'étape fermentaire. Les produits de fermentation peuvent inclure, par exemple, des alcools et des acides

Par exemple, différents brevets décrivent des souches capables de produire les produits d'intérêt cités précédemment et ce à partir de gaz de synthèse. Parmi les souches acétogènes du genre *Clostridium,* on peut citer le brevet US5173429 décrivant une souche de *Clostridium Ijungdahlii* (ATCC 49587) produisant de l'éthanol et de l'acétate. D'autres souches de la même espèce décrites dans les documents WO 2000/68407, EP117309, les brevets US5173429, US5593886 et US6368819, WO1998/00558 et WO2002/08438. Certaines souches *de Clostridium* comme les souches de *Clostridium autoethanogemim* (DSM 10061 et DSM 19630) décrites dans les documents WO2007/117157 et WO2009/151342, *Clostridium ragsdalei* (P11, ATCC BAA-622) décrites dans le brevet US7704723 ou encore *Clostridium carboxidivorans* (ATCC PTA-7827) décrits dans la demande de brevet US2007/0276447, sont elles aussi capables de produire par fermentation des molécules d'intérêt à partir de gaz (H₂, CO et/ou CO₂)

Le ou lesdits microorganismes ou souches acétogènes utilisés dans l'étape fermentaire du procédé selon l'invention sont de préférence choisies parmi les microorganismes suivants : *Acetogenium kivui, Acetoanaerobium noterae, Acetobacterium woodii, Alkalibaculum bacchi* CP11 (ATCC BAA-1772), *Blautia producta, Butyribacterium methylotrophicum, Caldanaerobacter subterraneous, Caldanaerobacter pacificus subterraneous, hydrogenoformans Carboxydothermus, Clostridium aceticum, Clostridium acetobutylicum, Clostridium acetobutylicum* P262, *Clostridium autoethanogenum* (DSM 10061 de DSMZ Allemagne), *Clostridium autoethanogenum* (DSM 23693 de DSMZ Allemagne), *Clostridium autoethanogenum* (DSM 24138 de DSMZ Allemagne), *Clostridium carboxidivorans* P7 (ATCC PTA-7827), *Clostridium coskatii* (ATCC PTA-10522), *Clostridium drakei, Clostridium Ijungdahlii* PETC (ATCC 49587), *Clostridium ERI2 Ijungdahlii* (ATCC 55380), *Clostridium Ijungdahlii* C-01 (ATCC 55988), *Clostridium Ijungdahlii* O- 52 (ATCC 55889), *Clostridium magnum, Clostridium pasteurianum* (DSM 525 du DSMZ Allemagne), *Clostridium ragsdali* P11 (ATCC BAA-622), *Clostridium scatologenes, Clostridium thermoaceticum, Clostridium ultunense, Desulfotomaculum kuznetsovii, Eubacterium limosum, sulfurreducens Geobacter, Methanosarcina acetivorans, Methanosarcina Barken, Morrella thermoacetica, Morrella thermoautotrophica, Oxobacter pfennigii, Peptostreptococcus productus, Ruminococcus productus, Thermoanaerobacter kivui,* et des mélanges de ceux-ci.

En outre, il doit être entendu que d'autres microorganismes capables d'assimiler l'H₂, le CO (autrement appelés carboxydotrophes) et/ou le CO₂ en tant que source de carbone peuvent également être utilisée dans l'étape e) de la présente invention. L'ensemble des microorganismes cités précédemment sont dits anaérobies, c'est-à-dire incapable de croître en présence d'oxygène. Mais des microorganismes aérobies peuvent également être employés tel que des microorganismes appartenant à l'espèce *Escherichia coli.* Une publication a par exemple démontré la possibilité de réaliser une souche génétiquement modifiée pour exprimer les gènes codant pour les enzymes responsables de l'assimilation du CO (gènes de la voie métabolique de Wood-Ljungdahl afin de produire des molécules d'intérêt, tel que l'isopropanol, à partir d'un intermédiaire métabolique important : l'acetyl CoA (Trawick, J.D.; Burk, M.J.; Burgard, A.P. Microorganisms and Methods for Conversion of Syngas and Other Carbon Sources to Useful Products. Patent WO2010/071697). Burk, M.; Schilling, C.H.; Burgard, A.; Trawick, J.D. Methods and Organisms for Utilizing Synthesis Gas or Other Gaseous Carbon Sources and Methanol. Patent WO2009/094485). D'autres microorganismes génétiquement modifiés ont été décrits pour produire de l'isopropanol comme par exemple le microorganisme *C. Ijungdhalii* (US2012/0252083 ; Lanzatech)

Dans des modes de réalisation préférés, les microorganismes sont choisies parmi *Clostridium autoethanogenum, Clostridium Ijungdahlii, Clostridium aceticum, Morella thermoacetica, Acetobacterium woodi* et *Alkalibaculum bacchi* pour la production d'éthanol et/ou d'acétate, *Clostridium autoethanogenum, Clostridium Ijungdahlii* et *C. ragdalei* pour la production de 2,3 Butanediol et *Clostridium carboxidivorans, Clostridium drakei, Clostridium scatologenes* ou *Butyribacterium methylotrophicum* pour la production de butyrate et de butanol.
Des cultures comprenant un mélange de deux ou plusieurs micro-organismes peuvent être également utilisés.

L'étape fermentaire e) peut avantageusement être mise en œuvre de manière aérobies ou anaérobies et de préférence de manière anaérobie.

L'étape fermentaire e) est avantageusement mise en œuvre dans un ou plusieurs réacteurs ou « bioréacteurs ».
Le terme «bioréacteur» comprend un dispositif de fermentation consistant en une ou plusieurs cuves ou réacteurs à tubes, comprenant les dispositifs de type CSTR ou Continuous Stirred Tank Reactor selon la terminologie anglo-saxonne, ICR ou Immobilized Cell Reactor selon la terminologie anglo-saxonne, TBR ou Trickle Bed Reactor, fermenteur de type Gas Lift, colonnes à bulle, ou réacteur à membrane tels que le système HFMBR ou Hollow Fibre membrane Bio-Reactor selon la terminologie anglo-saxonne, un mélangeur statique, ou tout autre dispositif approprié pour le contact gaz-liquide.

La partie dudit effluent de purge de l'étape d) comprenant du monoxyde de carbone (CO), et du dioxyde de carbone (CO₂) utilisée comme charge de l'étape fermentaire e) est amenée dans le ou les réacteurs de fermentation sous la forme d'un substrat gazeux. Chaque réacteur de fermentation contient un milieu de culture.
De préférence, la concentration requise en substrat gazeux issu de la purge (CO, CO₂, H₂) dans le milieu de culture du ou desdits réacteurs de fermentation est d'au moins 2,5 mmol/L de CO et/ou CO₂.
La partie dudit effluent de purge de l'étape d) utilisée comme charge de l'étape fermentaire e) est amenée dans le ou les réacteurs de fermentation sous la forme d'un substrat gazeux contenant avantageusement une teneur importante de CO, de préférence une teneur comprise entre 2 et 100% en poids de CO, de manière préférée une teneur comprise entre 20% et 100% en poids de CO, de manière très préférée entre 30% et 95% en poids de CO, de manière plus préférée entre 35% et 80% en poids de CO, et de manière encore plus préférée entre 40% et 60% en poids de CO.
De préférence, la partie dudit effluent de purge de l'étape d) utilisée comme charge de l'étape fermentaire d) sous forme de substrat gazeux présente avantageusement un ratio massique H₂/CO compris entre 0 et 1,2 et de préférence entre 0,5 et 1,2 et de manière très préférée entre 0,5 et 1,1 ou H₂/CO₂ compris entre 0 et 1,7 et de préférence entre 1 et 1,7 et très préférée entre 1,2 à 1,6.

Selon un mode de réalisation préféré, l'étape fermentaire e) comprend une chaîne de propagation d'une souche acétogène afin de fournir une quantité suffisante de cellules pour inoculer un ou plusieurs réacteurs principaux, ladite chaîne de propagation comprenant : i) l'inoculation de la souche acétogène dans un premier réacteur de propagation fournissant une densité minimale de cellules viables pour un second réacteur de propagation ayant un volume plus important, et ii) la croissance de ladite souche acétogène dans le second réacteur pour fournir une densité de cellules adaptées pour inoculer un troisième réacteur de propagation, le plus important en terme de volume. Si besoin, la chaîne de propagation peut comprendre une nombre de réacteurs de propagation plus important.
L'étape fermentaire comprend également une étape de production dans lequel le processus fermentaire est optimal, c'est-à-dire dans laquelle les molécules d'intérêt dont produites en grande quantité. Le flux comprenant au moins un composé oxygéné tel que revendiqué est donc produit dans ladite étape de production.

De préférence, l'étape de propagation peut être mise en œuvre dans un ou plusieurs réacteurs de propagation du microorganisme, l'ensemble de ces réacteurs étant connecté pour permettre le transfert de la culture microbienne.
Un ou plusieurs réacteurs de « production » dans lequel le processus fermentaire se réalise sont également mis en œuvre.
Les microorganismes ou souches acétogènes sont généralement cultivées jusqu'à ce qu'une densité cellulaire optimale soit obtenue pour inoculer les réacteurs de production. Ce taux d'inoculum peut varier de 0,5 à 75 % ce qui permet d'avoir des réacteurs de production plus grands que les réacteurs de propagation. Ainsi le réacteur de propagation peut être utilisé pour ensemencer plusieurs autres réacteurs de production plus grands.
Dans le cas où l'étape fermentaire comprend une étape de propagation et une étape de production, l'effluent gazeux comprenant du monoxyde de carbone (CO), et du dioxyde de carbone (CO₂) peut avantageusement être introduite dans l'étape fermentaire au niveau des réacteurs de l'étape de production.
Au moins un substrat carboné additionnel peut avantageusement être utilisé en association avec les substrats gazeux issus de l'étape b) de séparation, pour faire croître les microorganismes dans l'étape de propagation. Ledit substrat carboné peut avantageusement être choisi parmi les n monosaccharides tels que le glucose, le fructose ou le xylose, les polysaccharides tels que l'amidon, le saccharose, le lactose ou la cellulose, les intermédiaires métaboliques comme le pyruvate ou tout autre substrat carboné connu de l'homme de l'art comme pouvant être assimilés par les microorganismes utilisés dans le procédé. Ledit substrat carboné peut également être un mélange de deux ou plusieurs de ces substrats carbonés.

La maitrise des conditions opératoires est également nécessaire pour optimiser la mise en œuvre de l'étape fermentaire. A titre d'exemple, Lowe et al (Microbiological Review, 1993 57 : 451-509), ou Henstra et al (Current Opinion in Biotechnology 2007, 18:200-206), résument les conditions opératoires optimales en terme de températures et de pH, pour faire croître les microorganismes utilisables dans le procédé fermentaire. Le pH est un des facteurs les plus importants pour l'activité fermentaire des microorganismes employés dans le procédé. De préférence, ladite étape fermentaire d) est mise en œuvre à un pH compris entre 3 et 9, de préférence entre 4 et 8, et de manière plus préféré entre 5 et 7,5.

La température est également un paramètre important pour améliorer la fermentation car il influence aussi bien l'activité microbienne que la solubilité des gaz utilisés comme substrat. Le choix de la température dépend du microorganisme utilisé, certaines souches étant capables de croitre dans des conditions de températures modérées (souches dites mésophiles) et d'autres dans des conditions de températures élevées (microorganismes thermophiles). De préférence, ladite étape fermentaire e) est mise en œuvre à une température de croissance comprise entre 20 et 80°C. De préférence, ladite étape fermentaire c) est mise en œuvre à une température de croissance comprise entre 20 et 40°C pour les souches mésophiles et de manière préférée entre 25 et 35 et entre 40 et 80°C pour les souches thermophiles et de manière préférée entre 50 et 60 °C.

Le potentiel d'oxydo-réduction (autrement dit potentiel « redox ») est également un paramètre important à maitriser dans le procédé fermentaire. Le potentiel redox est de préférence inférieur à -450 mV et de préférence compris entre -150 et -250 mV.
Ladite étape fermentaire c) est par ailleurs avantageusement mise en œuvre à une pression comprise entre 0,1 et 0,4 MPa.

Le milieu nutritif ou milieu de culture de l'étape fermentaire peut avantageusement contenir au moins un agent réducteur de manière à améliorer les performances du processus fermentaire par le contrôle du potentiel redox de l'étape fermentaire.

Le milieu nutritif peut également comprendre des minéraux, des vitamines, des co-facteurs métalliques ou des métaux spécifiques aux metalloenzymes impliqués dans les voies de conversion du gaz en produits d'intérêt. Des milieux nutritifs anaérobies appropriées pour la fermentation de l'éthanol en utilisant le CO et/ou le CO₂ en tant que seule(s) source(s) de carbone sont connus de l'homme de l'art. Par exemple, des milieux appropriés sont décrits dans les brevet US5173429 et US5593886 et WO02/08438, WO2007/115157 et WO2008/ 115080 ou la publication J.R. Phillips et al. (Bioresource Technology 190 (2015) 114-121).

La composition du milieu nutritif doit permettre une conversion efficace du substrat gazeux en molécule d'intérêt. Cette conversion est avantageusement au minimum de 5 % et elle peut aller jusqu'à 99%, de préférence de 10 à 90 %, et de manière préférée 40 à 70%.
Le milieu nutritif peut contenir au moins une ou plusieurs d'une source d'azote, une ou plusieurs sources de phosphore et une ou plusieurs d'une source de potassium. Le milieu nutritif peut comprendre l'un de ces trois composés, ou toute combinaison des trois, et dans un aspect important, le milieu doit comprendre les trois composés. La source d'azote peut-être choisie parmi le chlorure d'ammonium, le phosphate d'ammonium, le sulfate d'ammonium, le nitrate d'ammonium, et des mélanges de ceux-ci. La source de phosphore peut-être choisie parmi l'acide phosphorique, le phosphate d'ammonium, le phosphate de potassium, et des mélanges de ceux-ci. La source de potassium peut peut-être choisie parmi le chlorure de potassium, le phosphate de potassium, le nitrate de potassium, le sulfate de potassium, et des mélanges de ceux-ci.

Le milieu nutritif peut également comprendre un ou plusieurs métaux comme du fer, du tungstène, du nickel, du cobalt, du magnésium, du soufre et de la thiamine. Le milieu peut comprendre un quelconque de ces composants, ou toute combinaison et dans un aspect important, comprend l'ensemble de ces composants. La source de fer peut-être choisie parmi le chlorure ferreux, le sulfate ferreux et leurs mélanges. La source de tungstène peut être choisi parmi le tungstate de sodium, tungstate de calcium, le tungstate de potassium et leurs mélanges. La source de nickel peut inclure une source de nickel choisi dans le groupe constitué par le chlorure de nickel, sulfate de nickel, nitrate de nickel, et des mélanges de ceux-ci. La source de cobalt peut être choisi parmi le chlorure de cobalt, du fluorure de cobalt, le bromure de cobalt, l'iodure de cobalt et des mélanges de ceux-ci. La source de magnésium peut être choisi parmi le chlorure de magnésium, du sulfate de magnésium, le phosphate de magnésium, et des mélanges de ceux-ci. La source de soufre peut comprendre la cystéine, le sulfure de sodium, et des mélanges de ceux-ci.

L'étape fermentaire e) peut également avantageusement être mise en œuvre comme décrit dans les demandes de brevet WO2007/117157, WO2008/115080, US6340581, US6136577, US 5593886, US5807722 et US5821111.
Comme mentionné précédemment, les conditions opératoires optimales pour effectuer cette étape fermentaire dépendent en partie du ou des microorganisme(s) utilisé(s). Les paramètres les plus importants à contrôler comprennent la pression, la température, le débit de gaz et de liquide, le pH du milieu, le potentiel redox, la vitesse d'agitation et le niveau d'inoculum. Il faut aussi s'assurer que les teneurs en substrats gazeux dans la phase liquide ne soit pas limitatifs. Des exemples de conditions opératoires convenables sont décrites dans les brevets WO02/08438, WO07/117157 et WO08/115080. Le ratio entre l'H₂ et les substrats gazeux CO et CO₂ peut également être important pour contrôler la nature des alcools produits par les microorganismes fermentaires. Dans la demande de brevet WO12/131627, il est par exemple décrit qu'en fonction du taux d'hydrogène il est possible de produire soit de l'éthanol seul, soit de l'éthanol et du 2,3 butanediol si le % d'H₂ est inférieur à 20% (volume). La composition typique du gaz de purge alimentant les réacteurs de production, permettrait avantageusement de produire par du 2,3 butanediol et de l'éthanol par *Clostridium autoethanogenum.*

De manière préférée, la fermentation doit être réalisée à une pression supérieure à la pression ambiante. Une pression supérieure à la pression ambiante permet d'augmenter substantiellement le taux de transfert de gaz dans la phase liquide afin qu'il soit assimilé par le microorganisme en tant que source de carbone. Ce fonctionnement permet notamment une réduction du temps de rétention (définie comme le volume de liquide dans le bioréacteur divisé par le débit d'écoulement de gaz d'entrée) dans le bioréacteur et donc une meilleure productivité (définie comme le nombre de grammes de molécules d'intérêt produites par litre et par jour de production) du procédé fermentaire. Des exemples d'amélioration de la productivité sont décrits dans le brevet WO02/08438.

Le flux liquide fermentaire comprend au moins un flux comprenant au moins un composé oxygéné choisi parmi l'éthanol, le n-propanol, l'isopropanol, le butanol, l'isobutanol, l'hexanol, l'acide butyrique, l'acide hexanoique, l'acide lactique, l'acétone, le butanone et le 2,3-butylène glycol (butane-2,3-diol), seuls ou en mélange.

Ledit flux liquide fermentaire produit par l'étape fermentaire e) contient également avantageusement du milieu nutritif, des molécules d'intérêt (alcools, alcools acides, acides), c'est-à-dire un flux de composés oxygénés tel que décrit ci-dessus et des cellules bactériennes.

Un appoint d'hydrogène peut avantageusement être introduit ou non dans ladite étape fermentaire c) dans le cas où la composition de la charge alimentant ladite étape ne comprend pas une quantité suffisante d'hydrogène. L'utilisation d'un substrat gazeux pauvre en H₂ entraine une production d'acides importantes. En effet, comme mentionné précédemment, l'apport d'hydrogène supplémentaire permet d'améliorer la conversion du CO présents dans le milieu fermentaire en alcools (selon les équations-bilans de Bertsch and Müller Biotechnol Biofuels (2015) 8:210 : 6 CO → 1 éthanol + 4 CO₂ puis 6 H₂ + 2 CO₂ → 1 éthanol + 0,3 ATP) et de favoriser la conversion du CO₂.

L'hydrogène d'appoint peut avantageusement provenir de tout procédé permettant la production d'hydrogène, tel que par exemple d'un procédé de reformage à la vapeur ou d'un procédé de reformage catalytique, de l'électrolyse de l'eau, de la déshydrogénation des alcanes, et sa pureté en hydrogène est le plus souvent comprise entre 75 et 99,9 % volume.

### Etape f) de séparation optionnelle

Ledit flux liquide fermentaire produit par l'étape fermentaire e) est avantageusement séparé dans une étape f) de manière à obtenir ledit flux de composés oxygénés.

Le procédé selon l'invention comprend éventuellement une étape f) de séparation dudit flux fermentaire obtenu à l'issue de l'étape e) en au moins ledit flux comprenant au moins un composé oxygéné, une fraction aqueuse, et un effluent gazeux non réagi.
De préférence, la fraction aqueuse séparée comprend majoritairement de l'eau.
De préférence, l'effluent gazeux non réagi comprenant des gaz incondensables et de préférence du monoxyde de carbone et du dioxyde de carbone qui n'ont pas réagi lors de l'étape de fermentation e).
Ladite étape de séparation est avantageusement mise en œuvre par des méthodes de séparation connues de L'homme du métier.
Dans un mode de réalisation préféré, l'étape f) de séparation peut avantageusement être réalisée avec de la vapeur d'eau selon les techniques connues de l'Homme du métier.
En particulier, ladite séparation est avantageusement réalisée en utilisant de la vapeur d'eau provenant de l'étape a) de de pyrolyse catalytique.
La vapeur d'eau peut avantageusement être générée par la combustion du coke et du char dans le régénérateur et récupérée via un refroidisseur de catalyseur chaud ou « cat-cooler » selon la terminologie anglo-saxonne.
Dans ce mode de réalisation, l'énergie nécessaire à la séparation est donc fournie par l'étape de pyrolyse catalytique ce qui renforce la synergie entre les deux étapes du procédé.

Ledit flux de composés oxygénés produit par ladite étape fermentaire et séparé dudit flux liquide fermentaire dans l'étape f) de séparation, peut également avantageusement être séparé en différents flux comprenant lesdites molécules d'intérêt.
A titre d'exemple, il est possible de récupérer les différents alcools produits contenus dans ledit flux de composés oxygénés par des procédés tels que la distillation ou l'évaporation fractionnée. Des exemples de ce type de procédés comprennent ceux décrits dans WO2007/117157, WO2008/115080, US6340581, US6136577, US5593886, US5807722 et US5821111.
La distillation d'alcool à partir dudit flux de liquide fermentaire issu de ladite étape e) peut avantageusement être réalisée après passage sur une colonne à bière permettant d'obtenir un flux d'alcools concentrés et le recyclage éventuel des acides et autres molécules réassimilables par le ou les microorganismes employé(s) dans ladite étape de fermentation. Le flux d'alcools peut ensuite être traité via diverses colonnes de distillation permettant la séparation sélective ou non des différents alcools présents. Pour ce faire, il est possible d'utiliser des techniques de déshydratation d'alcools, via notamment des tamis moléculaire, bien connues de l'homme de l'art.

L'étape e) fermentaire permet donc l'obtention d'un flux de composés oxygénés tel que décrit ci-dessus, ledit flux, avantageusement séparé de la souche, comprenant entre 0,1 et 99,9 % d'eau et entre 99,9 et 0,1 % d'alcools, de diols et/ou d'acides carboxyliques et de préférence entre 30 et 98% d'eau et entre 2 et 70% d'alcools, de diols et/ou d'acides carboxyliques.
Selon un mode de réalisation très préféré de l'invention, aucun flux issu de l'étape fermentaire e) n'est recyclé dans ladite étape de pyrolyse catalytique a) et de préférence ledit flux de composés oxygénés n'est pas recyclé dans l'étape a) de pyrolyse catalytique.

### Etape g) de recycle optionnelle

Le procédé comprend éventuellement une étape g) de recycle dans l'étape a) de pyrolyse catalytique d'au moins une partie dudit effluent gazeux non réagi issu de l'étape e) de fermentation et éventuellement séparé dans l'étape f) de séparation optionnelle.
L'effluent gazeux non réagi issu de l'étape f) de séparation optionnelle du flux fermentaire comprenant avantageusement du monoxyde de carbone et du dioxyde de carbone.
La composition dudit effluent gazeux non réagi est identique à la composition de la partie dudit effluent de purge de l'étape d) utilisée comme charge de l'étape fermentaire e).
De préférence, l'effluent gazeux non réagi issu de l'étape f) comprend une teneur en monoxyde de carbone comprise entre 2 et 100% en poids de CO, de manière préférée une teneur comprise entre 20% et 100% en poids de CO, de manière très préférée entre 30% et 95% en poids de CO, de manière plus préférée entre 35% et 80% en poids de CO, et de manière encore plus préférée entre 40% et 60% en poids de CO, les pourcentages étant exprimés en pourcentage massique par rapport à la masse totale de l'effluent.
De préférence, l'effluent gazeux non réagi issu de l'étape f) présente avantageusement un ratio massique H₂/CO compris entre 0 et 1,2 et de préférence entre 0,5 et 1,2 et de manière très préférée entre 0,5 et 1,1 ou H₂/CO₂ compris entre 0 et 1,7 et de préférence entre 1 et 1,7 et très préférée entre 1,2 à 1,6.

L'effluent gazeux non réagi issu de l'étape f) de séparation optionnelle du flux fermentaire comprend également avantageusement de l'hydrogène, des gaz légers en C1-C4, des oléfines et peut également contenir une faible teneur en impuretés aromatiques telles que par exemple en benzène et en toluène. La somme de la teneur en éléments composant ledit effluent est égale à 100%.

De préférence, ledit effluent gazeux non réagi dans l'étape fermentaire et éventuellement séparé dans l'étape f) est recyclée, de préférence via un compresseur, dans l'étape a) de pyrolyse catalytique. Ce flux gazeux sert alors de fluide d'entrainement de la charge dans ledit réacteur.
Par ailleurs, l'effluent gazeux incondensable non réagi dans l'étape de fermentation et recyclé dans l'étape de pyrolyse catalytique présente une pression partielle d'hydrocarbure plus élevé par rapport à l'art antérieur ce qui permet d'améliorer la production de BTX dans l'étape de pyrolyse catalytique .

### Description des figures :

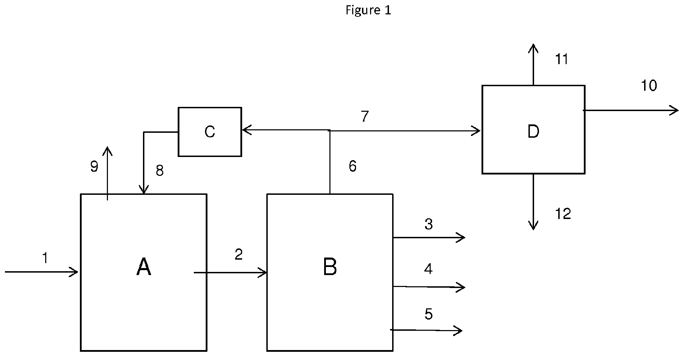

La figure 1 illustre le procédé selon l'invention dans le mode de réalisation préféré dans lequel le flux de composés oxygénés produit dans l'étape fermentaire n'est pas recyclé dans l'étape de pyrolyse catalytique.
Sur la figure 1, la biomasse est introduite via la conduite 1 dans un réacteur de pyrolyse catalytique opérant en lit fluidisé A. L'effluent gazeux de pyrolyse catalytique est ensuite envoyé via la conduite 2 dans une section de fractionnement B de manière à récupérer un effluent gazeux, incondensable, comprenant au moins du monoxyde de carbone (CO), et du dioxyde de carbone (CO₂) via la conduite 6, une coupe liquide dite BTX via la conduite 3, une coupe liquide lourde comprenant majoritairement des composés présentant un nombre d'atomes de carbone supérieur à 9, via la conduite 4 et de l'eau via la conduite 5.
Des fumées de combustion sont également soutirées du réacteur de pyrolyse via la conduite 9.
Au moins une partie dudit effluent gazeux comprenant du monoxyde de carbone (CO), et du dioxyde de carbone (CO₂) est recyclée via un compresseur C, dans le réacteur de l'étape de pyrolyse catalytique via la conduite 8.
Une purge dudit effluent gazeux de recycle est réalisée via la conduite 7 en amont du compresseur C.
La purge dudit effluent gazeux de recycle est ensuite envoyée via la conduite 7 dans une étape de fermentation D produisant un flux liquide fermentaire comprenant au moins un flux comprenant au moins un composé oxygéné soutiré via la conduite 10. L'étape fermentaire D comprend également la séparation du flux de liquide fermentaire obtenu en un flux comprenant au moins un composé oxygéné soutiré via la conduite 10, de l'eau soutirée via la conduite 12 et un flux gazeux d'incondensable comprenant du CO et du CO₂ non réagis, soutiré via la conduite 11.

L'invention est illustrée par les exemples suivants qui ne présentent, en aucun cas, un caractère limitatif.

### Exemple 1 : comparatif : pyrolyse catalytique sans introduction d'un flux de composés oxygénés.

L'exemple 1 présente le cas d'une pyrolyse catalytique d'une variété de pin d'une capacité de 2500 tonnes jour avec un recycle dans le réacteur de pyrolyse catalytique d'une partie de l'effluent gazeux, incondensable comprenant au moins du CO et CO₂ séparée de l'effluent gazeux issu de la pyrolyse. La biomasse est introduite dans le réacteur de pyrolyse catalytique à raison de 104 tonnes par heures. Le ratio massique recycle/biomasse est de 1,5 afin d'être dans les conditions hydrodynamiques souhaitées.

Dans cet exemple le catalyseur utilisé est une ZSM5 commerciale présentant une teneur en cristaux de 40%. Le réacteur est opéré à une température de 580°C, sous pression de 0,2 MPa abs et sous une VVH catalytique de 0,3 h-1.

Dans ces conditions le rendement en BTX est de 15% poids par rapport à la charge sèche hors cendres.

### Exemple 2 : selon l'invention

L'exemple 2 correspond au cas d'une pyrolyse catalytique menée dans les mêmes conditions opératoires que celles de l'exemple 1 mais pour lequel la purge de l'effluent gazeux, incondensable comprenant au moins du CO et CO₂ est envoyée à une unité de fermentation.

La purge en question constitue la charge de l'étape de fermentation et correspond à un flux de substrat gazeux de 33 tonnes par heure présentant la composition présenté dans le tableau 1.

**Tableau 1 : Composition de la purge constituant la charge de l'unité de fermentation L'étape de fermentation est menée à l'aide d'une souche de Clostridium Iungdahlii permettant spécifiquement la conversion du CO en éthanol dans les conditions opératoires suivantes :**

| Composition du gaz de recycle | % poids |
|---|---|
| Hydrogène | 0,5% |
| CO | 50,0% |
| CO₂ | 35,4% |
| Méthane | 7,3% |
| Ethane | 0,5% |
| Ethylène | 4,8% |
| Propane | 0,1% |
| Propylène | 1,3% |

Le pourcentage de CO contenu dans le substrat gazeux arrivant dans le procédé fermentaire est de 50% en poids et le milieu de croissance du microorganisme est le milieu PETC (American Type Culture Collection (ATCC) medium 1754).
L'étape de fermentation est alimentée par le flux de substrat gazeux décrit ci-dessus et est conduite à pression atmosphérique, avec une agitation de 300 rpm, à une température de 39°C , à un pH régulé entre 5,5 et 6,0 et à un potentiel redox de -250 mV. Il comprend une première phase de production du microorganisme au travers d'une chaine de propagation aboutissant à une quantité suffisante de microorganismes permettant d'inoculer les réacteurs de production.
Le procédé de production aboutit à la genèse d'un flux liquide fermentaire séparé de la souche extraite du réacteur, ledit liquide fermentaire comprenant 94% en poids d'eau 5% poids d'éthanol et 1 % poids d'acide acétique résiduel. Les alcools, principalement l'éthanol, contenus dans ce flux sont récupérés par distillation aboutissant à une coupe azéotropique comprenant 95% d'alcools.

Ainsi, une production totale de 8,5 tonnes d'éthanol par heures est générée ce qui correspond à un rendement d'environ 8%poids par rapport à la biomasse sèche hors cendre. Selon ce mode de l'invention, un produit à haute valeur ajoutée est généré en plus de la coupe BTX et ce à partir du CO destiné au final à être brûlé ce qui améliore très significativement la rentabilité globale du système.

### Exemple 3 : selon l'invention

L'exemple 3 correspond au cas d'une pyrolyse catalytique menée dans les mêmes conditions opératoires que celles de l'exemple 1 mais pour lequel la purge de l'effluent gazeux, incondensable comprenant au moins du CO et CO₂ est envoyée à une unité de fermentation spécifique permettant la conversion du CO en éthanol et en 2,3 butanediol.

La purge en question correspond à un flux de substrat gazeux de 33 tonnes par heure présentant la même composition que dans l'exemple 3 et présentée dans le tableau 1.
Par rapport à l'exemple 3, l'étape de fermentation est menée à l'aide d'une souche de *Clostridium autoethanogeum* DSMZ 10061 permettant spécifiquement la conversion du CO en éthanol et en 2,3 butanediol dans les conditions opératoires suivantes :
Le pourcentage de CO contenu dans le substrat gazeux arrivant dans le procédé fermentaire est de 50%, et le % d'Hydrogène de 0,5%. Le milieu de croissance est défini comme suit :
Milieu de croissance et de production d'alcools : Par litre de milieu
Solution 1 (MgCl₂.6H₂O 10 g/L, CaCl₂. 2H₂O 15 g/L) : 8,33 mL
Solution 2 (NaCL 12 g/L, KCI 15 g/L) : 8,33 mL
CH₃COONH₄ 3,00 g
solution de Resazurine (1g/L) : 1,00 mL
H₃PO₄ (85%) : 0,37 mL
Solution métaux 1 : 1 mL
Solution métaux 2 : 1mL
Solution de tungstate de sodium (2,94 g/L) : 0,1 mL
Solution de vitamines : 10,00 mL
Composition des solutions de métaux et vitamines
Solution métaux 1 (Par litre)
FeSO₄.7H₂O : 0,10 g
ZnSO₄.7H₂O : 0,20 g
NiCl₂. 6H₂O : 0,02 g
HCI (38%) : 30 mL
Solution métaux 2 (Par litre)
MnSO4. H₂O : 0,5 g
CoCl₂. 6H₂O : 0,5 g
H₃BO₃ : 0,3 g
Na MoO₄. 2H₂O : 0,03g
Na₂SeO₃ : 0,02 g
HCI (38%) : 5 mL
Solution de vitamines (Par litre)
Biotine : 20 mg
Acide folique : 20 mg
Pyridoxine : 10 mg
Thiamine : 50 mg
Riboflavine : 50 mg
Vitamine B3 : 50 mg
Acide pantothénique : 50 mg
Vitamine B12: 50 mg
Para-aminobenzoate : 50 mg
Acide lipoïque : 50 mg

L'étape de fermentation est alimentée par le flux de substrat gazeux décrit ci-dessus et est conduite en pression atmosphérique, à une température de 37°C, un pH régulé à 5,3 par du NH₄OH, un potentiel redox maintenu à -250 mV et un apport en souffre fourni par des ajouts de Na₂S. Il comprend une première phase de production du microorganisme au travers d'une chaine de propagation aboutissant à une quantité suffisante de microorganismes permettant d'inoculer les réacteurs de production.
Le procédé de production aboutit à la genèse d'un flux liquide fermentaire séparé de la souche extraite du réacteur, ledit liquide fermentaire comprenant 95% en poids d'eau 4,2% poids d'éthanol et 0,8 % poids de 2,3 butanediol. Ces alcools sont éventuellement récupérés par distillation aboutissant à une coupe azéotropique comprenant 95% d'alcools.
Ainsi, une production totale de 6,9 tonnes d'éthanol et de 1,4 tonnes de 2, 3 butanediol par heures est générée ce qui correspond à un rendement d'environ 6,6% et 1,3% poids par rapport à la biomasse sèche hors cendre respectivement pour l' éthanol et le 2,3 butanediol.

Comme pour l'exemple 2, la production de produits additionnels à valeur ajoutée à partir du CO destiné à la combustion améliore très significativement la rentabilité globale du système. Selon cet autre mode de l'invention, la production d'alcools peut être différente en fonction du mode de réalisation du procédé fermentaire permettant d'être plus flexible et d'être au plus proche de l'optimum économique.

## Revendications

1. Procédé de production de BTX et d'alcools à partir de biomasse comprenant au moins les étapes suivantes :
a) la pyrolyse catalytique de ladite biomasse dans un réacteur en lit fluidisé produisant un effluent gazeux de pyrolyse;
b) la séparation dudit effluent gazeux de pyrolyse en au moins une fraction BTX et un effluent gazeux comprenant au moins du monoxyde de carbone et du dioxyde de carbone,
c) le recycle d'au moins une partie dudit effluent gazeux comprenant au moins du monoxyde de carbone et du dioxyde de carbone dans le réacteur de ladite étape a),
d) la purge dudit effluent gazeux recyclé selon l'étape c) pour produire un effluent de purge,
e) l'envoi d'au moins une partie dudit effluent de purge de l'étape d) dans une étape de fermentation produisant un flux liquide fermentaire comprenant au moins un flux comprenant au moins un composé oxygéné choisi parmi les l'éthanol, le n-propanol, l'isopropanol, le butanol, l'isobutanol, l'hexanol, l'acide butyrique, l'acide hexanoique, l'acide lactique, l'acétone, le butanone et le 2,3-butylène glycol (butane-2,3-diol), seuls ou en mélange.

2. Procédé selon la revendication 1 dans lequel l'étape a) de pyrolyse catalytique opère en présence d'un catalyseur zéolithique comprenant au moins une zéolithe choisie parmi la ZSM-5, la ferriérite, la zéolithe Béta, la zéolithe Y, la mordenite, la ZSM-23, la ZSM-57, la EU-1, la ZSM-11, dopée ou non par un métal choisi parmi le Fer, le Gallium, le Zinc et le Lanthane.

3. Procédé selon l'une des revendications 1 ou 2 dans lequel l'étape a) de pyrolyse catalytique est mise en œuvre à une température comprise entre 400 et 1000°C, a une pression absolue comprise entre 0,1 et 0,5 MPa et à une vvh comprise entre 0,01 et 10 h⁻¹.

4. Procédé selon l'une des revendications 1 à 3 dans lequel ladite étape fermentaire e) est mise en œuvre en présence d'au moins un microorganisme choisi parmi les microorganismes suivants : Acetogenium kivui, Acetoanaerobium noterae, Acetobacterium woodii, Alkalibaculum bacchi CP11 (ATCC BAA-1772), Blautia producta, Butyribacterium methylotrophicum, Caldanaerobacter subterraneous, Caldanaerobacter paci*f*icus subterraneous, hydrogeno*f*ormans Carboxydothermus, Clostridium aceticum, Clostridium acetobutylicum, Clostridium acetobutylicum P262, Clostridium autoethanogenum (DSM 10061 de DSMZ Allemagne), Clostridium autoethanogenum (DSM 23693 de DSMZ Allemagne), Clostridium autoethanogenum (DSM 24138 de DSMZ Allemagne), Clostridium carboxidivorans P7 (ATCC PTA-7827), Clostridium coskatii (ATCC PTA-10522), Clostridium drakei, Clostridium Ijungdahlii PETC (ATCC 49587), Clostridium ERI2 Ijungdahlii (ATCC 55380), Clostridium Ijungdahlii C-01 (ATCC 55988), Clostridium Ijungdahlii O- 52 (ATCC 55889), Clostridium magnum, Clostridium pasteurianum (DSM 525 du DSMZ Allemagne), Clostridium ragsdali P11 (ATCC BAA-622), Clostridium scatologenes, Clostridium thermoaceticum, Clostridium ultunense, Desul*f*otomaculum kuznetsovii, Eubacterium limosum, sul*f*urreducens Geobacter, Methanosarcina acetivorans, Methanosarcina Barken, Morrella thermoacetica, Morrella thermoautotrophica, Oxobacter p*f*ennigii, Peptostreptococcus productus, Ruminococcus productus, Thermoanaerobacter kivui, et des mélanges de ceux-ci.

5. Procédé selon la revendication 4 dans lequel les microorganismes sont choisis parmi Clostridium autoethanogenum, Clostridium Ijungdahlii, Clostridium aceticum, Morella thermoacetica, Acetobacterium woodi et Alkalibaculum bacchi pour la production d'éthanol et/ou d'acétate, Clostridium autoethanogenum, Clostridium Ijungdahlii et C. ragdalei pour la production de 2,3 Butanediol et Clostridium carboxidivorans, Clostridium drakei, Clostridium scatologenes ou Butyribacterium methylotrophicum pour la production de butyrate et de butanol, des cultures comprenant un mélange de deux ou plusieurs micro-organismes peuvent être également utilisés.

6. Procédé selon l'une des revendications 1 à 5 dans lequel ladite étape fermentaire e) est mise en œuvre à une température de croissance comprise entre 20 et 80°C, à une pression absolue comprise entre 0,1 et 0,4 MPa et à un pH compris entre 3 et 9.

7. Procédé selon l'une des revendications 1 à 6 dans lequel un appoint d'hydrogène peut avantageusement être introduit ou non dans ladite étape fermentaire e).

8. Procédé selon l'une des revendications 1 à 7 dans lequel une étape f) de séparation dudit flux fermentaire obtenu à l'issue de l'étape e) en au moins ledit flux comprenant au moins un composé oxygéné, une fraction aqueuse, et un effluent gazeux non réagi est mise en œuvre.

9. Procédé selon la revendication 8 dans lequel ladite étape f) de séparation est réalisée en utilisant de la vapeur d'eau provenant de l'étape a) de pyrolyse catalytique.

10. Procédé selon l'une des revendications 8 ou 9 dans lequel ledit flux de composés oxygénés séparé à l'issue de l'étape f) n'est pas recyclé dans l'étape a) de pyrolyse catalytique.

## Patentansprüche

1. Verfahren zur Herstellung von BTX und von Alkoholen ausgehend von Biomasse, wobei es mindestens die folgenden Schritte umfasst:
a) katalytische Pyrolyse der Biomasse in einem Wirbelschichtreaktor, wobei ein gasförmiger Pyrolysestoffstrom erzeugt wird;
b) Auftrennen des gasförmigen Pyrolysestoffstroms in mindestens eine BTX-Fraktion und einen gasförmigen Stoffstrom, der zumindest Kohlenmonoxid und Kohlendioxid umfasst,
c) Rückführen zumindest einer Teilmenge des gasförmigen Stoffstroms, welcher zumindest Kohlenmonoxid und Kohlendioxid umfasst, in den Reaktor des Schrittes a),
d) Ablassen des gasförmigen Stoffstroms, welcher gemäß dem Schritt c) zurückgeführt wurde, um einen Ablass-Stoffstrom zu erzeugen,
e)Einleiten zumindest einer Teilmenge des Ablass-Stoffstroms des Schrittes d) in einen Fermentationsschritt, wobei ein flüssiger fermentationsbezogener Stoffstrom erzeugt wird, der mindestens einen Stoffstrom umfasst, welcher mindestens eine sauerstoffhaltige Verbindung umfasst, die aus Ethanol, n-Propanol, Isopropanol, Butanol, Isobutanol, Hexanol, Buttersäure, Hexansäure, Milchsäure, Aceton, Butanon und 2,3-Butylenglykol (Butan-2,3-diol), jeweils allein oder in Mischung, ausgewählt ist.

2. Verfahren nach Anspruch 1, wobei der Schritt a) der katalytischen Pyrolyse in Gegenwart eines zeolithartigen Katalysators betrieben wird, der mindestens einen Zeolith umfasst, welcher aus ZSM-5, Ferrierit, Zeolith Beta, Zeolith Y, Mordenit, ZSM-23, ZSM-57, EU-1, ZSM-11 ausgewählt ist, wobei er möglicherweise mit einem Metall dotiert ist, das aus Eisen, Gallium, Zink und Lanthan ausgewählt ist.

3. Verfahren nach einem der Ansprüche 1 oder 2, wobei der Schritt a) der katalytischen Pyrolyse bei einer Temperatur im Bereich von 400 bis 1.000 °C, bei einem Absolutdruck Bereich von 0,1 bis 0,5 MPa und bei einer stundenbezogenen Raumgeschwindigkeit im Bereich von 0,01 bis 10 Std.⁻¹ durchgeführt wird.

4. Verfahren nach einem der Ansprüche 1 bis 3, wobei der fermentationsbezogene Schritt e) in Gegenwart mindestens eines Mikroorganismus durchgeführt wird, der aus den folgenden Mikroorganismen ausgewählt ist: Acetogenium kivui, Acetoanaerobium noterae, Acetobacterium woodii, Alkalibaculum bacchi CP11 (ATCC BAA-1772), Blautia producta, Butyribacterium methylotrophicum, Caldanaerobacter subterraneous, Caldanaerobacter pacificus subterraneous, hydrogenoformans Carboxydothermus, Clostridium aceticum, Clostridium acetobutylicum, Clostridium acetobutylicum P262, Clostridium autoethanogenum (DSM 10061 der DSMZ Deutschland), Clostridium autoethanogenum (DSM 23693 der DSMZ Deutschland), Clostridium autoethanogenum (DSM 24138 der DSMZ Deutschland), Clostridium carboxidivorans P7 (ATCC PTA-7827), Clostridium coskatii (ATCC PTA-10522), Clostridium drakei, Clostridium ljungdahlii PETC (ATCC 49587), Clostridium ERI2 ljungdahlii (ATCC 55380), Clostridium ljungdahlii C-01 (ATCC 55988), Clostridium ljungdahlii O- 52 (ATCC 55889), Clostridium magnum, Clostridium pasteurianum (DSM 525 der DSMZ Deutschland), Clostridium ragsdali P11 (ATCC BAA-622), Clostridium scatologenes, Clostridium thermoaceticum, Clostridium ultunense, Desulfotomaculum kuznetsovii, Eubacterium limosum, sulfurreducens Geobacter, Methanosarcina acetivorans, Methanosarcina Barken, Morrella thermoacetica, Morrella thermoautotrophica, Oxobacter pfennigii, Peptostreptococcus productus, Ruminococcus productus, Thermoanaerobacter kivui sowie den Mischungen derselben.

5. Verfahren nach Anspruch 4, wobei die Mikroorganismen zur Erzeugung von Ethanol und/oder von Acetat aus Clostridium autoethanogenum, Clostridium ljungdahlii, Clostridium aceticum, Morella thermoacetica, Acetobacterium woodi und Alkalibaculum bacchi, zur Erzeugung von 2,3-Butandiol aus Clostridium autoethanogenum, Clostridium ljungdahlii und C. ragdalei sowie zur Erzeugung von Butyrat und von Butanol aus Clostridium carboxidivorans, Clostridium drakei, Clostridium scatologenes oder Butyribacterium methylotrophicum ausgewählt sind, wobei auch Kulturen verwendet werden können, die eine Mischung aus zwei oder mehreren Mikroorganismen umfassen.

6. Verfahren nach einem der Ansprüche 1 bis 5, wobei der fermentationsbezogene Schritt e) bei einer Wachstumstemperatur im Bereich von 20 bis 80 °C, bei einem Absolutdruck im Bereich von 0,1 bis 0,4 MPa und bei einem pH-Wert im Bereich von 3 bis 9 durchgeführt wird.

7. Verfahren nach einem der Ansprüche 1 bis 6, wobei im fermentationsbezogenen Schritt e) vorteilhafterweise zusätzlicher Wasserstoff eingeleitet werden kann, aber nicht muss.

8. Verfahren nach einem der Ansprüche 1 bis 7, wobei ein Schritt f) durchgeführt wird, in welchem der fermentationsbezogene Stoffstrom, wie er nach Abschluss des Schrittes e) erhalten wurde, zumindest in den Stoffstrom, welcher mindestens eine sauerstoffhaltige Verbindung umfasst, in eine wässrige Fraktion sowie in einen gasförmigen Stoffstrom aufgetrennt wird, welcher keine Umsetzung erfahren hat.

9. Verfahren nach Anspruch 8, wobei der Trennschritt f) durchgeführt wird, indem Wasserdampf verwendet wird, welcher aus dem Schritt a) der katalytischen Pyrolyse stammt.

10. Verfahren nach einem der Ansprüche 8 oder 9, wobei der Stoffstrom aus sauerstoffhaltigen Verbindungen, wie er nach Abschluss des Schrittes f) abgetrennt wurde, nicht in den Schritt a) der katalytischen Pyrolyse zurückgeführt wird.

## Claims

1. Process for producing BTX and alcohols from biomass, comprising at least the following steps:
a) catalytic pyrolysis of said biomass in a fluidized-bed reactor producing a gaseous pyrolysis effluent,
b) separation of said gaseous pyrolysis effluent into at least a BTX fraction and a gaseous effluent comprising at least carbon monoxide and carbon dioxide,
c) recycling at least part of said gaseous effluent comprising at least carbon monoxide and carbon dioxide into the reactor of said step a),
d) purging said gaseous effluent recycled according to step c) to produce a purge effluent,
e) sending at least part of said purge effluent from step d) into a fermentation step producing a liquid fermentation stream comprising at least one stream comprising at least one oxygenated compound chosen from ethanol, n-propanol, isopropanol, butanol, isobutanol, hexanol, butyric acid, hexanoic acid, lactic acid, acetone, butanone and 2,3-butylene glycol (2,3-butanediol), alone or as a mixture.

2. Process according to Claim 1, in which the catalytic pyrolysis step a) takes place in the presence of a zeolite catalyst comprising at least one zeolite chosen from ZSM-5, ferrierite, zeolite beta, zeolite Y, mordenite, ZSM-23, ZSM-57, EU-1 and ZSM-11, whether or not doped with a metal chosen from iron, gallium, zinc and lanthanum.

3. Process according to either of Claims 1 and 2, in which the catalytic pyrolysis step a) is performed at a temperature of between 400 and 1000°C, at an absolute pressure of between 0.1 and 0.5 MPa and at an HSV of between 0.01 and 10 h-¹.

4. Process according to one of Claims 1 to 3, in which said fermentation step e) is performed in the presence of at least one microorganism chosen from the following microorganisms: Acetogenium kivui, Acetoanaerobium noterae, Acetobacterium woodii, Alkalibaculum bacchi CP11 (ATCC BAA-1772), Blautia producta, Butyribacterium methylotrophicum, Caldanaerobacter subterraneous, Caldanaerobacter pacificus subterraneous, hydrogenoformans Carboxydothermus, Clostridium aceticum, Clostridium acetobutylicum, Clostridium acetobutylicum P262, Clostridium autoethanogenum (DSM 10061 from DSMZ Germany), Clostridium autoethanogenum (DSM 23693 from DSMZ Germany), Clostridium autoethanogenum (DSM 24138 from DSMZ Germany), Clostridium carboxidivorans P7 (ATCC PTA-7827), Clostridium coskatii (ATCC PTA-10522), Clostridium drakei, Clostridium ljungdahlii PETC (ATCC 49587), Clostridium ERI2 ljungdahlii (ATCC 55380), Clostridium ljungdahlii C-01 (ATCC 55988), Clostridium ljungdahlii O-52 (ATCC 55889), Clostridium magnum, Clostridium pasteurianum (DSM 525 from DSMZ Germany), Clostridium ragsdali P11 (ATCC BAA-622), Clostridium scatologenes, Clostridium thermoaceticum, Clostridium ultunense, Desulfotomaculum kuznetsovii, Eubacterium limosum, sulfurreducens Geobacter, Methanosarcina acetivorans, Methanosarcina Barken, Morrella thermoacetica, Morrella thermoautotrophica, Oxobacter pfennigii, Peptostreptococcus productus, Ruminococcus productus, Thermoanaerobacter kivui, and mixtures thereof.

5. Process according to Claim 4, in which the microorganisms are chosen from Clostridium autoethanogenum, Clostridium ljungdahlii, Clostridium aceticum, Morella thermoacetica, Acetobacterium woodii and Alkalibaculum bacchi for producing ethanol and/or acetate, Clostridium autoethanogenum, Clostridium ljungdahlii and C. ragdalei for producing 2,3-butanediol and Clostridium carboxidivorans, Clostridium drakei, Clostridium scatologenes or Butyribacterium methylotrophicum for producing butyrate and butanol; cultures comprising a mixture of two or more microorganisms may also be used.

6. Process according to one of Claims 1 to 5, in which said fermentation step e) is performed at a growth temperature of between 20 and 80°C, at an absolute pressure of between 0.1 and 0.4 MPa and at a pH of between 3 and 9.

7. Process according to one of Claims 1 to 6, in which a hydrogen supplement may advantageously be optionally introduced into said fermentation step e).

8. Process according to one of Claims 1 to 7, in which a step f) of separating said fermentation stream obtained on conclusion of step e) into at least said stream comprising at least one oxygenated compound, an aqueous fraction and an unreacted gaseous effluent is performed.

9. Process according to Claim 8, in which said separation step f) is performed using steam originating from the catalytic pyrolysis step a).

10. Process according to either of Claims 8 and 9, in which said stream of oxygenated compounds separated out on conclusion of step f) is not recycled into the catalytic pyrolysis step a).
